(19)

Europäisches
Patentamt
European
Patent Office
Office européen
des brevets

(11) **EP 3 081 633 A1**

(12) **EUROPEAN PATENT APPLICATION**

(43) Date of publication:
**19.10.2016 Bulletin 2016/42**

(51) Int Cl.:
***C12M 1/32*** *(2006.01)*        ***C12M 1/00*** *(2006.01)*

(21) Application number: **16165441.3**

(22) Date of filing: **14.04.2016**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**MA MD**

(30) Priority: **16.04.2015 JP 2015084524**

(71) Applicant: **ARKRAY, Inc.
Minami-ku
Kyoto-shi,
Kyoto 601-8045 (JP)**

(72) Inventors:
• **HIRANO, Kunio
Kyoto-shi, Kyoto 602-0008 (JP)**
• **NODA, Yuichiro
Kyoto-shi, Kyoto 602-0008 (JP)**

(74) Representative: **Golding, Louise Ann
Dehns
St Bride's House
10 Salisbury Square
London EC4Y 8JD (GB)**

(54) **DEVICE AND SYSTEM FOR CELL CULTURE**

(57)    A cell culture device comprising:
a channel having an inlet end for the introduction of a liquid and a bottom surface, wherein said channel is configured for the flow through of said liquid; and
a plurality of recesses for containing cells, which are formed on said bottom surface of said channel;
wherein said plurality of recesses are closely arranged on said bottom surface of said channel.

EP 3 081 633 A1

## Description

TECHNICAL FIELD

The present invention relates to a cell culture device and a cell culture system.

BACKGROUND ART

As a therapeutic method for diabetes, especially type I diabetes, pancreas transplantation or pancreatic islet transplantation is effective. However, in such transplantation, there are problems such as a limited number of organs donated, and the necessity of administration of an immunosuppressive agent for prevention of immune rejection. On the other hand, studies on induction of differentiation of pluripotent stem cells such as induced pluripotent stem cell (iPS cells) into pancreatic islet cells have been widely carried out using cells derived from mouse or human.

US 8859286 B shows a method for induction of differentiation into endodermal cells, especially pancreatic islet cells, using stimulating factors such as TGF-β. However, although this document shows stimulating factors necessary for induction of the cell fate into endodermal lineages, especially pancreatic islet cells, whether the induced cell population is kept to have a flat structure or formed into a three-dimensional aggregate is not shown, and no method for controlling the size of the cell population in this process is shown.

JP 2011-115161 A shows a method for culturing embryonic stem cells (ES cells) or iPS cells on a matrix composed of positively-charged, nano-sized fibers or particles, to induce pancreatic islet cells from pluripotent stem cells without using feeder cells. However, there is neither description nor suggestion on formation of aggregates.

DIABETES, VOL. 61, AUGUST 2012, 2016-2029 shows a method in which human pluripotent stem cells are induced to differentiate into pancreatic islet cells, and the differentiated pancreatic islet cells are allowed to form cell aggregates, to prepare pseudoislets. The document also shows that transplantation of the pseudoislets ameliorated the diabetic condition of diabetic model mice. In this document, the differentiation induction was performed by adherent culture of the cells on the bottom surface of a culture dish, and, during the process, cell populations were detached from the bottom surface of the culture dish, and subjected to nonadherent culture (suspension culture) to allow formation of the aggregates. However, since the formation of the aggregates depends of random adhesion of cell populations to each other during the suspension culture, the size of each aggregate cannot be controlled.

JP 5039715 B shows a method and a device in which seeded cells are allowed to form aggregates in recesses constituted by a non-cell-adhesive hydrogel substrate. However, this document does not show a method of their application to differentiation induction from pluripotent stem cells. There are also known methods for inducing differentiation into islet cells using cells in a state of aggregates formed by shake culture, such as the methods described in PLoS ONE., VOL. 7, May 2012, e37004, Thomas C. Schulz et al.; and Cell. VOL. 159, Oct 2014, 428-439, Felicia W Pagliuca et al.

JP 2007-135593 A discloses a method for forming cell aggregates in a culture dish the surface of which is modified such that cell adhesion is inhibited, and mentions the cell seeding density and the serum concentration in the medium as factors which largely contribute to the size of each aggregate. The document describes a method in which the area where cell adhesion may occur on the bottom surface of the culture dish is restricted to limit the size of the resulting cell aggregate. However, this document does not show a method of its application to differentiation induction.

SUMMARY OF THE INVENTION

An object of the present invention is to provide a cell culture device and cell culture system suitable for culturing cells with efficient formation of cell aggregates. When some kinds of cells are induced to differentiation, it is sometimes necessary to form cell aggregates, and it is preferable to control the size of the cell aggregates to a uniform size because nutrients required for cell growth and oxygen are supplied to the center portion of the cell aggregates by diffusion. US 8859286 B discloses stimulation factors required for inducing cell fates to endoderm lineage, especially pancreatic islet cells but does not disclose a method of controlling the size of cell aggregates. Further, cell aggregates are formed randomly by cell-cell contact and thus it is difficult to control the size of cell aggregates, and it is necessary to precipitate cells by centrifugation at the time of medium change in the methods disclosed in DIABETES, VOL. 61, AUGUST 2012, 2016-2029, PLoS ONE., VOL. 7, May 2012, e37004, and Cell. VOL. 159, Oct 2014,428-439. JP 5039715 B does not disclose a method of exchanging medium in a simple manner. Therefore, the present invention aims to provide a technique for efficiently culturing cell aggregates.

In order to efficiently culturing cells by forming cell aggregates, the following cell culture device can be used. However, the usage of the cell culture device and cell culture system described below are not limited to producing pseudoislet, and these the cell culture device and cell culture system can be used for culturing various kinds of cells which can be cultured in the form of cell aggregates.

The first embodiment of the cell culture device of the present invention is a cell culture device comprising:

a channel having an inlet end for the introduction of a liquid and a bottom surface, wherein the channel is configured for the flow through of said liquid; and a plurality of recesses for containing cells, which are formed on the bottom surface of the channel;

wherein

the plurality of recesses are closely arranged on the bottom surface of the channel.

According to the first embodiment, the number of seeded cells can be controlled, and a single cell aggregate can be prepared in each recess (that is, in a state where cell aggregates are not in contact with each other). In addition, sequential replacement of the medium can be carried out in a state where the cell aggregates are retained in the wells (recesses). Since the plurality of recesses are closely arranged, production of cell aggregates in areas other than the recesses can be suppressed, and the medium can be almost uniformly supplied to the plurality of recesses. Therefore, aggregates with almost uniform size can be prepared.

The second embodiment of the cell culture device of the present invention is the cell culture device as described above, wherein
the channel has a discharge end for discharging the liquid;
the channel comprises a first area the width of which increases from the inlet end to a predetermined length;
the first area has two side-wall surfaces which are perpendicular to the bottom surface of the channel; and
the angle formed by each tangent plane of the two side-wall surfaces and the direction from the inlet end toward the discharge end is not more than 45°.

According to the second embodiment, the liquid can be uniformly supplied to the recesses. Therefore, aggregates with almost uniform size can be formed in the recesses.

The third embodiment of the cell culture device of the present invention is the cell culture device as described above, wherein
the area of the plurality of recesses on the bottom surface of the channel is not less than half of the area of the bottom surface.

According to the third embodiment, production of cell aggregates in areas other than the recesses can be suppressed. Therefore, aggregates with almost uniform size can be formed in the recesses.

The fourth embodiment of the cell culture device of the present invention is the cell culture device as described above, wherein
the flow rate of the medium introduced to the channel is not more than 10 cm/s.

According to the fourth embodiment, fresh medium can be uniformly supplied to the cells contained in the recesses. Therefore, aggregates with almost uniform size can be formed in the recesses.

The fifth embodiment of the cell culture device of the present invention is the cell culture device as described above, wherein each of said recces has a rim and the rim of the recess is chamfered.

According to the fifth embodiment, cells are less likely to accumulate in areas other than the recesses, so that cell aggregates are less likely to be produced in the areas other than the recesses. Therefore, aggregates with almost uniform size can be efficiently formed, and loss of cell aggregates can be reduced.

The sixth embodiment of the cell culture device of the present invention is the cell culture device as described above, wherein
the width of the recess is 100 $\mu$m to 5 mm; and
the height of the recess is 100 $\mu$m to 5 mm.

According to the sixth embodiment, almost uniform cell aggregates having a predetermined size can be formed in the recesses.

An embodiment of the cell culture system of the present invention is a cell culture system comprising:

any one of the cell culture devices as described above;
a feeding device for adjusting the amount of a liquid introduced to the cell culture device; and
a storage device for storing cells and a medium discharged from the cell culture device.

According to this embodiment, the medium can be introduced into the cell culture device at a predetermined rate. Therefore, almost uniform cell aggregates having a predetermined size can be formed in the recesses.

According to the present invention, a technique which enables culture of almost uniform cell aggregates can be provided.

BRIEF DESCRIPTION OF THE DRAWINGS

Fig. 1 is a diagram showing an embodiment of the cell culture system of the present invention.
Fig. 2 is an exploded perspective view showing an example of the the cell culture device.
Fig. 3 is a diagram showing an example of the cell culture device of the present invention.
Fig. 4 is a diagram showing an example of the cross-sectional view of the cell culture device along the X1-X1 line in Fig. 3.
Fig. 5 is a diagram showing an example of the cross-sectional view of the cell culture device along the Y1-Y1 line in Fig. 3.
Fig. 6 is a diagram showing examples of the cross-sectional shape of a plurality of wells.
Fig. 7 is a diagram showing examples of the cross-sectional shape of the well.
Fig. 8 is a diagram showing examples the shape of wells on the bottom surface portion.
Fig. 9 is a diagram showing examples of the shape of the channel.

Fig. 10 is a cross-sectional view of the cell culture device showing a modification example of the cover.

Fig. 11 is a diagram showing an example of arrangement of the wells.

Fig. 12 is a diagram showing a modification example of the cell culture device of the present invention.

Fig. 13 is a diagram showing an alternate example 1 of the cell culture system of the present invention.

Fig. 14 is a diagram showing an alternate example 2 of the cell culture system of the present invention.

EMBODIMENTS FOR CARRYING OUT THE INVENTION

<Embodiments of Cell Culturing Device and Cell Culturing System>

Embodiments are described below with reference to the drawings. The constitutions of the embodiments are merely examples, and the constitution of the present invention is not limited to the specific constitutions of the embodiments.

As an embodiment of the present invention, a cell culture system comprising a cell culture device for forming cell aggregates is described below. The aggregates formed using the cell culture system are used for, for example, induction of differentiation into islet cells. However, the cell culture system according to the present embodiment can be used for formation of aggregates for induction of differentiation into various cells including islet cells, by selecting the type(s) of the medium/media used for stimulating the cells depending on the type of the differentiation induction of interest.

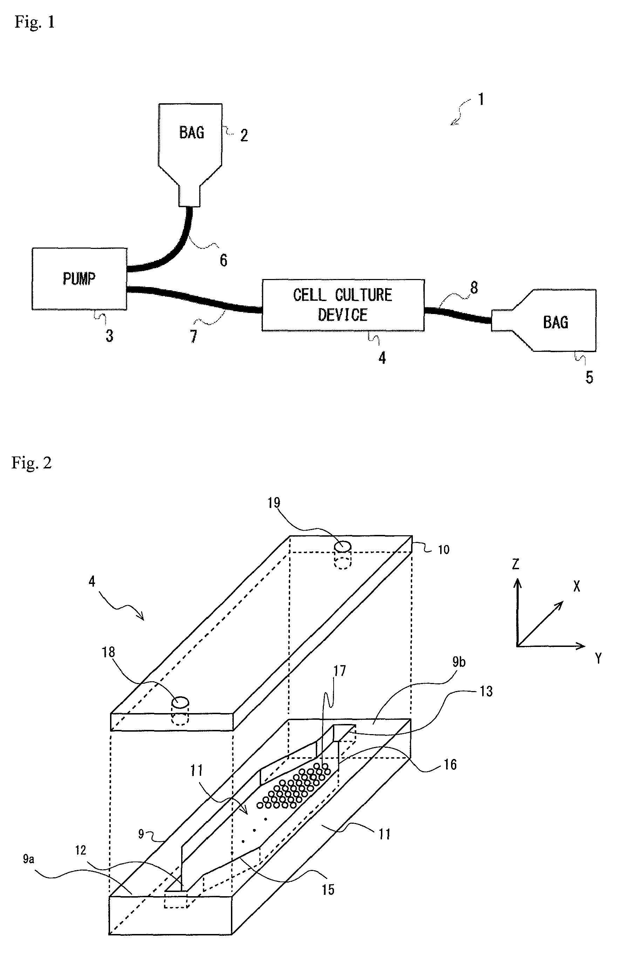

Fig. 1 is a diagram showing an example of the cell culture system of the present embodiment. The cell culture system 1 shown in Fig. 1 comprises a bag 2, pump 3, cell culture device 4, and waste liquid storage bag 5. The bag 2 is connected to the pump 3 through a tube 6; the pump 3 is connected to the cell culture device 4 through a tube 7; and the cell culture device 4 is connected to the bag 5 through a tube 8.

The bag 2 contains a medium to be supplied to the cell culture device 4.

The medium is a liquid containing oxygen and nutrients necessary for the survival of the cells and/or a substance which gives stimulation for differentiation of the cells into desired cells. As the medium, a medium containing a plurality of cells to be cultured in the cell culture device 4 may be used. A medium containing cells is also called a cell suspension. The term "medium" is used hereinafter without distinguishing a medium and a cell suspension. Instead of the medium, a liquid other than a medium, for example, physiological saline, may be used. A plurality of bags 2 are provided, and these bags 2 contain a plurality of types of media or cell suspensions whose components are modified depending on the cell culture conditions. Each bag 2 is replaced at an appropriate timing.

For example, when the cells are seeded in the cell culture device 4, the bag 2 is replaced with one containing a cell suspension. The bag 2 is formed using, for example, a resin. Instead of the bag 2, a container such as a bottle capable of storing the medium may be used. The bag 2, and the container capable of storing the medium, are examples of the storage means.

The pump 3 is placed between the bag 2 and the cell culture device 4. The medium sucked from the bag 2 through the tube 6 is discharged into the tube 7 by the pump 3. The amount of the medium discharged by the pump 3 per unit time can be controlled, and, by controlling the discharge rate, the amount of the medium introduced into the cell culture device 4 (flow) can be controlled. The discharge of the medium by the pump 3 can be stopped and the flow rate becomes zero temporarily. The pump 3 may be, for example, a tubing pump. Other examples of the pump 3 include rotary pumps and gear pumps. The pump 3 is an example of the feeding device. Alternatively, a constitution in which the pump 3 is arranged between the cell culture device 4 and the bag 5, and the medium from the bag 2 is drawn into the cell culture device 4, may be applied. As the bag 2 and the pump 3, a syringe pump in which a bag for storing the medium and a pump for discharging the medium are integrated, or the like may be used.

The cell culture device 4 has a channel having an inlet end and a discharge end. The cells and the medium are introduced from the inlet end, and discharged from the discharge end. The inlet end is connected to the pump 3 through piping such as a tube. The discharge end is connected to the bag 5 through piping such as a tube. The channel has a plurality of wells (culture wells) in which cells are to be contained and cultured. The wells are an example of the recesses. The cell culture device 4 is described later in detail.

The waste liquid storage bag 5 is connected to the discharge end of the cell culture device 4 through the tube 8, and stores (collects) the medium discharged from the discharge end of the cell culture device 4. As the bag 5, a resin bag may be used. Instead of the bag 5, another kind of container such as a resin bottle capable of storing the medium may be used. The bag 5, and the container capable of storing the medium, are examples of the storage device.

The tube 6 is piping through which the bag 2 is connected to the pump 3.

An end of the tube 6 is connected to the bag 2, and the other end is connected to the pump 3. The tube 7 is piping through which the pump 3 is connected to the cell culture device 4. An end of the tube 7 is connected to the pump 3, and the other end is connected to the culture device 4. The tube 8 is piping through which the cell culture device 4 is connected to the bag 5. An end of the tube 8 is connected to the culture device 4, and the other end is connected to the bag 5. Examples of the tubes 6, 7, and 8 include resin tubes. As the tubes 6, 7, and 8, other kinds of piping capable of transferring the medium, such as resin pipes, may be used.

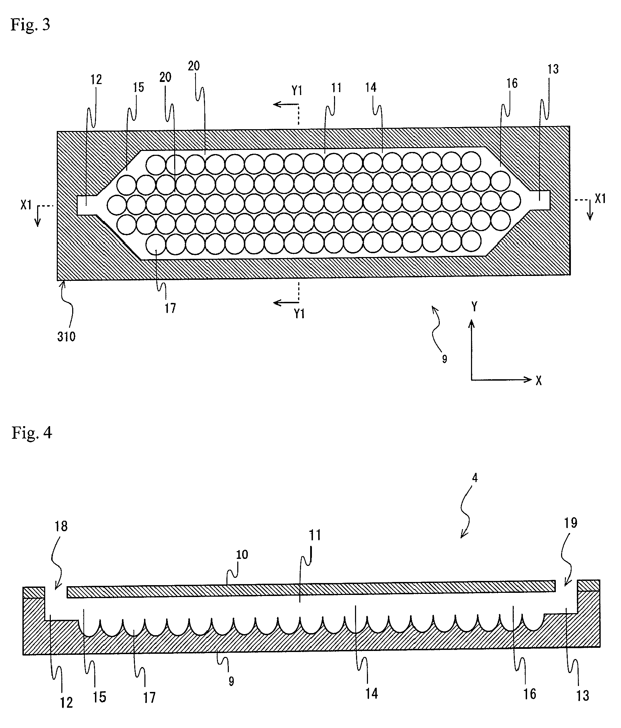

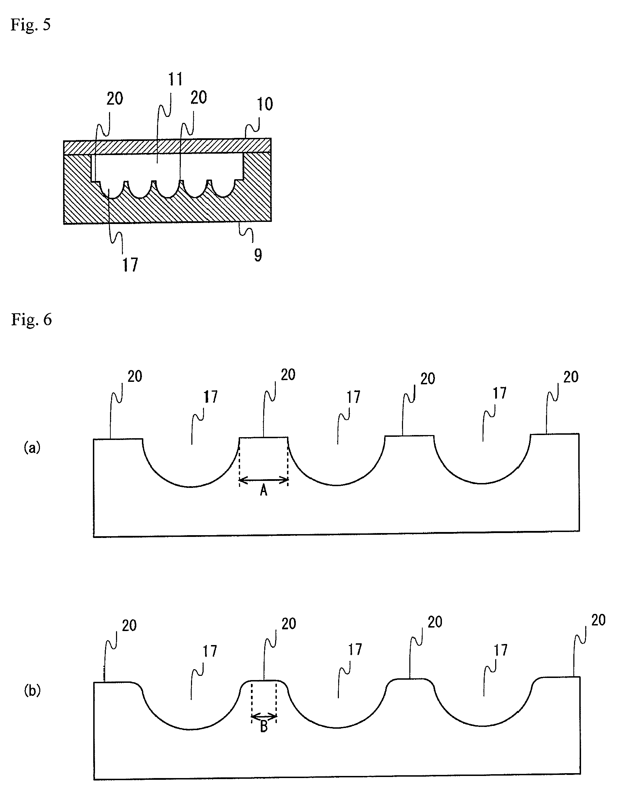

Fig. 2 is an exploded perspective view showing an example of the cell culture device. Fig. 3 is a top view of a main body 9 of the cell culture device 4. Fig. 4 is a diagram showing an example of the cross-sectional view of the cell culture device along the X1-X1 line in Fig. 3. Fig. 5 is a diagram showing an example of the cross-sectional view of the cell culture device along the Y1-Y1 line in Fig. 3. The constitution of the cell culture device 4 is described below using Fig. 2 to Fig. 5.

As shown in Fig. 2, the cell culture device 4 comprises a main body 9 and a cover 10. The main body 9 is formed to have a planar shape having a longitudinal direction (X-direction in Fig. 2), and a transverse direction (Y-direction in Fig. 2) perpendicular to the longitudinal direction. On the upper surface of the main body 9, a groove 11 is formed in the longitudinal direction. The groove 11 is formed by one end section (inlet end) 12 and the other end section (discharge end) 13; a middle section 14; a taper section 15; and a taper section 16. The one end section 12 is formed at one end section 9a of the main body 9, and the other end section 13 is formed at the other end section 9b of the main body 9. The middle section 14 is formed between the one end section 9a and the other end section 9b of the main body 9. Through the taper section 15, the one end section 12 is connected to the middle section 14. Through the taper section 16, the middle section 14 is connected to the other end section 13. The length of the width of the middle section 14 is not less than the width of the one end section 12 and the other end section 13. The width of the taper section 15 increases from the one end section 12 toward the middle section 14, and the width of the taper section 16 gradually decreases from the middle section 14 toward the other end section 13. The cell culture device 4 has a channel formed by the groove 11 which starts from the one end section 12 and passes through the taper section 15, middle section 14, and taper section 16, finally leading to the other end section. The taper section 15, middle section 14, and taper section 16 are examples of the first area, second area, and third area, respectively.

The bottom surface of the groove 11 is formed such that the one end section 12 to the other end section 13 are flush with each other, and a plurality of recesses 17 (which are also referred to as well 17) are formed as wells on the bottom surfaces of the taper section 15, middle section 14, and taper section 16. The wells 17 are arranged such that the distances between adjacent wells are small. The wells 17 are closely arranged not only on the middle section 14, but also on the taper section 15 and the taper section 16. In the present embodiment, the plurality of wells 17 on the taper section 15 and the taper section 16 are arranged such that a part of the wells are placed along the wall surfaces of the taper sections. The rows of the wells 17 in the flow direction are arranged such that the centers of the wells 17 are located at shifted positions between adjacent rows.

In the case shown in Fig. 3, the opening section of the well 17 has a circular shape in its planar view. As shown in the cross-sectional views in Fig. 4 and Fig. 5, the well 17 has a semielliptical shape in its longitudinal section. However, the shape of the well 17 is not limited thereto.

The well 17 has a size which enables formation of an aggregate having a predetermined size. In order to promote formation of an aggregate upon precipitation of cells into the bottom portion, the cross-sectional area of the plane parallel to the opening section of the well 17 decreases from the opening section toward the bottom portion of the well 17. The lower limit of the diameter of the well 17 is preferably 100 $\mu$m, more preferably 200 $\mu$m, or may be 400 $\mu$m. The upper limit of the diameter of the well 17 is preferably 5 mm, more preferably 3 mm, or may be 800 $\mu$m. The lower limit of the height (depth) of the well is preferably 100 $\mu$m, more preferably 200 $\mu$m, or may be 400 $\mu$m. The upper limit of the height (depth) of the well is preferably 5 mm, more preferably 1 mm, or may be 800 $\mu$m. The size of the well 17 includes the diameter, height, capacity, and the like of the well 17. The diameter of the well 17 is an example of the width of the well 17. The capacity of the well 17 is preferably 0.001 $\mu$l/well to 10 $\mu$l/well, more preferably 0.001 to 1 $\mu$l/well, or may be 0.005 to 0.1 $\mu$l/well.

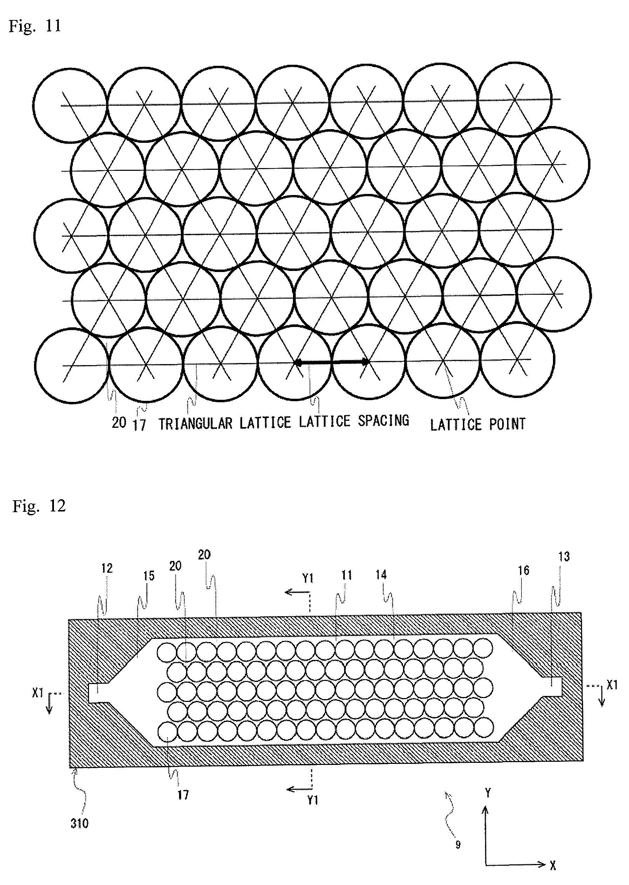

Fig. 11 is a diagram showing an example of arrangement of the wells. Fig. 11 shows a top view of the well 17. As shown in Fig. 11, for example, lattice points of a hypothetical equilateral triangular lattice are plotted on the bottom surfaces of the taper section 15, middle section 14, and taper section 16 of the groove 11. The lattice spacing of the equilateral triangular lattice is not less than the diameter of the well 17. Here, the center of the circle of each well 17 is arranged at the position of a lattice point of the equilateral triangular lattice. By this, the wells 17 can be uniformly arranged on the bottom surfaces of the taper section 15, middle section 14, and taper section 16 of the groove 11. In this embodiment, in cases where the lattice spacing of the equilateral triangular lattice is almost equal to the diameter of the well 17, the flat sections 20 on the bottom surface of the groove 11 (the portion where the well 17 is absent on the bottom surface of the groove 11) can be small, so that the wells 17 can be arranged such that they are closely adjacent to each other. The wells 17 are uniformly arranged on the bottom surfaces of the taper section 15, middle section 14, and taper section 16 of the groove 11 such that the wells are adjacent to each other. The arrangement of the wells 17 on the groove 11 is not limited to the arrangement shown in this figure. Alternatively, unlike the example shown in Fig. 11, the center of the circle of each well 17 may be arranged at the position of a lattice point of a square lattice having a lattice spacing which is not less than the diameter of the well 17. The arrangement of the wells 17 on the groove 11 may be either periodic as shown in Fig. 11, or aperiodic. On the bottom surfaces of the taper section 15, middle section 14, and taper section 16 of the groove 11, the area of the wells 17 is at least not less than half of the area of the bottom surface.

As shown in Fig. 3, the wall surfaces (side-wall surfaces) perpendicular to the bottom surface of the taper section 15 connect the one end section 12 to the middle section 14 through planes. The angle formed by each wall surface and the X-axis is not more than 45°, so that the medium introduced from the side of the one end section 12 reaches the wall surfaces. The same applies to the angle formed by each wall surface of the taper section 16 and the X-axis. By forming the wall surfaces of the taper section 15 and the taper section 16 at such an angle, the medium introduced into the channel can be allowed to flow in a state where the medium is uniformly spread in the channel.

Fig. 6 is a diagram showing examples of the cross-sectional shape of a plurality of wells. In the case of Fig. 6(a), flat sections 20 appear between wells 17, and in the vicinities of wells 17. In the cell culture, when cells in the form of a cell suspension are seeded in the cell culture device 4, some cells may be placed on flat sections 20 rather than in wells 17. In such a case, the sizes of the cell clusters prepared on the flat sections 20 on which the cells are placed may be different from the sizes of the cell clusters formed in wells 17. That is, the size of each aggregate formed in the cell culture device 4 may vary. In view of this, the area of the flat sections 20 in the vicinities of the wells 17 on the bottom surface of the groove 11 is preferably small. In such a case, aggregates are less likely to be produced in areas other than the inside of the wells 17.

In the case of Fig. 6(b), the rim (upper end) of each well 17 is chamfered. Therefore, the area of the plane of each flat section 20 in this case (for example, B in Fig. 6(b)) is smaller than the area of the plane of each flat section 20 in Fig. 6(a) (for example, A in Fig. 6(a)). In cases where the rim of each well 17 is chamfered as shown in Fig. 6(b), even if a cell is placed between wells 17, the cell is likely to be contained in (likely to fall into) one of the wells 17. That is, cells are less likely to be retained in areas other than the wells 17. Since, in such a case, cells are unlikely to be cultured in areas other than the wells 17, uniform cell aggregates are likely to be formed. By arranging as many wells 17 as possible on the bottom surfaces of the taper section 15, middle section 14, and taper section 16 of the groove 11, the area of the flat sections 20 can be reduced.

The cover 10 is a rectangular parallelepiped plate, and covers the groove 11 of the main body 9. In the cover 10, a first hole 18 and a second hole 19 are formed as penetrating holes. In a state where the cover 10 is attached to the main body 9, the first hole 18 makes the one end section 12 of the groove 11 open to the outside, and the second hole 19 makes the other end section 13 of the groove 11 open to the outside. To the first hole 18, the other end of the tube 7 is connected. To the second hole 19, the one end of the tube 8 is connected. The cover 10 may be detachably attached to the main body 9, or may be fixed to the main body 9.

By the attachment of the cover 10 to the main body 9, the first hole 18 and the second hole 19 are used as an inlet (inlet end) and an outlet (discharge end) for the medium, respectively. Since the inlet and the outlet for the medium are provided in the cover 10, the groove 11 of the main body 9 can be used as a channel. For example, a medium is introduced from the inlet end such that the channel is filled with the medium, and the medium which could not be contained in the channel overflows from the second hole 19, and is recovered into the bag 5 through the tube 8. Therefore, the upper surface of the main body 9 and the lower surface of the cover 10 are tightly bonded to each other using an adhesive or a sealant, or by bolting, to prevent leakage of the medium introduced into the channel to the outside. When the medium is introduced into the channel from the inlet, an air layer may be present in the upper portion of the channel. In such a case, the device is constituted such that, for example, a medium outlet is formed on a side surface of the main body 9 to allow overflow of the medium from the outlet when the liquid level in the groove 11 reaches a predetermined height.

Since nonadherent culture is carried out in the wells 17, the main body 9 and the cover 10 may have a culture surface subjected to non-adhesive treatment. However, the main body 9 and the cover 10 are preferably made of a material which allows cell culture in a nonadherent state. Such a material is preferably a non-cytotoxic hydrophilic material having a three-dimensional structure. The material is more preferably a transparent material from the viewpoint of enabling easy observation of the culture state. Since the cell culture device 4 may be used in a carbon dioxide atmosphere, the device is preferably made of a material permeable to carbon dioxide. More specifically, the material is preferably a hydrogel.

Examples of the material used for preparing the hydrogel include synthetic polymers that can form hydrogels, such as products prepared by chemical cross-linking or radiation cross-linking of synthetic polymers including polyvinyl alcohol, polyvinyl pyrrolidone, polyethylene glycol, poly-2-hydroxyethyl methacrylate, poly-2-hydroxyethyl acrylate, polyacrylamide, polyacrylic acid, and polymethacrylic acid; and products prepared by cross-linking of copolymers prepared with monomers constituting the polymers described above. Polysaccharides such as agarose, alginic acid, dextran, and cellulose as natural polymers, and derivatives thereof; and cross-linked products of proteins such as gelatin and albumin, and derivatives thereof; may also be used.

The main body 9 is prepared by, for example, using a metal mold having the shape of the main body 9 shown in Fig. 2 to prepare a resin mold made of PDMS (dimethylpolysiloxane) or the like, and then further transferring the shape into PDMS. The bottom surface of the main body 9 including the wells 17 (culture surface) is preferably subjected to non-adhesive treatment. The main body 9 and the cover 10 may be integrally molded using a 3D printer.

For each of the main body 9 and the cover 10, a transparent material is preferably used from the viewpoint of enabling easy observation of the cells (aggregates) in the wells 17 from the outside. In cases where the cells are observed by immunostaining, the material of each of the main body 9 and the cover 10 preferably does not show emission of fluorescence from the resin itself, that is, the so-called autofluorescence, from the viewpoint of observation of clear images. PDMS is an excellent material since it has high transparency due to its high light transmittance in the visible wavelength region and hardly emits autofluorescence. Since injection molding of PDMS has recently become possible, mass production of the main body 9 and the cover 10 is possible at low cost by using PDMS.

Examples of materials having properties similar to those of PDMS include cycloolefin polymers and cycloolefin copolymers. Examples of transparent materials which can be mass-produced by injection molding include acryl, polystyrene, polypropylene, polyethylene, polycarbonate, ABS resins, and glasses. These materials may be used as materials for the main body 9 and the cover 10.

In the main body 9, the bottom surface portion including the wells 17 (the surface portion of the bottom surface) may be prepared with a hydrogel, and the channel portion (the portion excluding the surface portion of the bottom surface) may be prepared with PDMS or the like. In such a case, the main body 9 is formed by, for example, fitting the bottom surface portion made of a hydrogel into the channel portion made of PDMS or the like. By forming the main body 9 in this way, drying of the hydrogel can be suppressed.

(Example of Use of Cell Culture Device and Cell Culture System)

An example of use of the cell culture system 1 of the present embodiment is described below. In the cell culture system 1, cell aggregates are formed and cultured. The cell culture device 4 is placed in an atmosphere of, for example, 5% carbon dioxide using a $CO_2$ incubator or the like. The cell culture device 4 is arranged such that the bottom surface is horizontally placed. In the beginning, the tube 7 is not connected to the cell culture device 4.

Seeding of the cells into the cell culture device 4 is carried out by directly introducing a cell suspension into the inlet end of the cell culture device 4 using a pipette or the like. The amount of the cell suspension introduced into the cell culture device 4 is larger than the capacity of the channel of the cell culture device 4. By the introduction of the cell suspension in an amount larger than the capacity of the channel of the cell culture device 4 into the cell culture device, the entire channel of the cell culture device 4 can be filled with the cell culture liquid. The excess cell suspension is discharged from the discharge end, passes through the tube 8, and then collected into the bag 5. The length of time during which the cell culture liquid is introduced into the cell culture device 4 is sufficiently shorter (for example, about 2 seconds) than the length of time during which the cell culture liquid is left to stand in the cell culture device 4. By the introduction of the cell culture liquid into the cell culture device 4 in a sufficiently short time, the cells can be uniformly spread among the wells 17.

The cell suspension can be introduced into the cell culture device 4 by its introduction into the upstream portion of the cell culture device 4. That is, the cell suspension may be introduced into the bag 2, may pass through the pump 3, and may then be introduced into the cell culture device 4. Alternatively, a port may be provided in the tube 7 or the cell culture device 4, and the cell suspension may be introduced from the port using a syringe.

The origin and the type of the cells used herein are not limited, and either eukaryotic cells or prokaryotic cells may be used. Cells derived from a mammal is preferred. Examples of the cells include pluripotent stem cells such as induced pluripotent stem (iPS) cells and embryonic stem (ES) cells. A cell suspension containing pluripotent stem cells is produced as follows. First, pluripotent stem cells cultured in an adherent state in advance are detached from the culture dish, and dissociated into single cells. The method for detaching the cells in this process is not limited, and an agent such as trypsin or EDTA may be used therefor. By suspending the pluripotent stem cells dissociated into single cells in a medium or the like at the cell density of interest, the cell suspension can be prepared.

When the cell suspension is introduced into the cell culture device 4, and then left to stand for a while (for example, 10 minutes to 1 hour), the cells contained in the cell suspension precipitate in the groove 11 of the cell culture device 4, and are stored in the wells 17. The length of time during which the cell suspension is left to stand is sufficiently longer than the length of time during which the cell suspension is introduced into the cell culture device 4. In this process, almost the same amounts of cells are contained in the wells 17 since the wells 17 have the same size. Since the device is formed such that the area of the flat sections 20 on the bottom surface of the groove 11 is small, cells are unlikely to precipitate in areas other than the wells 17. In the wells 17, the cells contact with each other, resulting in spontaneous formation of aggregates. The aggregates formed in the wells 17 are retained in the wells 17. The size of each aggregate can be controlled by changing the cell concentration in the cell suspension and the size of each well. By using wells 17 having uniform size, aggregates having uniform size can be formed.

The other end of the tube 7 is then connected to the inlet end of the cell culture device 4. A predetermined type of medium (hereinafter referred to as medium A) is placed in the bag 2 according to predetermined cell culture conditions, and the pump 3 is operated such that the flow of the medium A discharged becomes a predetermined flow (flow rate). The medium A discharged from the pump 3 is introduced into the cell culture device 4, and the medium A is supplied to the cells in the wells 17. A predetermined type of medium (hereinafter referred to as medium B) is further placed in the bag 2 according to predetermined cell culture conditions, and the pump 3 is operated such that the flow of the medium B discharged becomes a predetermined flow (flow rate). The medium B discharged from the pump 3 is introduced into the cell culture device 4, and the medium B is supplied to the cells in the wells 17. Here, during the introduction of the medium B into the cell culture device 4, the medium A, which is introduced in advance, is pushed by the later-introduced medium B, and discharged from the discharge end of the cell culture device 4. The medium discharged is collected into the bag 5. The time of the introduction of the medium and the amount of the medium to be introduced are determined based on the cell culture conditions. By this, the medium in the cell culture device 4 can be replaced from the medium A to the medium B. By the use of the cell culture device 4, replacement of the medium can be carried out without performing a complex operation such as centrifugation. If necessary, replacement of the medium in the cell culture device 4 can be further carried out in the same manner. It is also possible to continuously supply fresh medium to the cells in the wells 17 by continuously introducing the medium to the cell culture device 4. For example, by controlling the flow of the medium using the pump 3, the rate at which the medium flows (flow rate) through the channel of the cell culture device 4 is adjusted to not more than 10 cm/s, preferably not more than 1 cm/s. By this, fresh medium can be supplied to the cells. The introduction of the medium may be temporarily stopped, and the flow rate of the medium may be temporarily 0. In terms of the transfer of the medium, the medium is preferably introduced into the cell culture device 4 under conditions where a laminar flow is formed in a channel structure which is the same as that of the groove 11 except that the wells 17 are absent. Such conditions can be realized using the cell culture device 4 of the present embodiment. The laminar flow herein means that the streamlines of a fluid are parallel to the direction of the channel, and means a flow field which is not a turbulent flow. Conditions under which such a laminar flow can be realized are conditions where the Reynolds number (Re) is not more than 10, or, for example, conditions where the Reynolds number (Re) is not more than 1.

The Reynolds number is a dimensionless number given by the following Equation. In cases where Re<2320, the flow is regarded as a laminar flow (that is, no turbulent flow is generated).

$$ \mathrm{Re} \ = \frac{\rho \upsilon d}{\mu} = \frac{\upsilon d}{\nu} $$

In the equation, $\rho$ represents the density of the fluid (kg/m$^3$); $\upsilon$ (m/s) represents the mean velocity in the cross section of the pipe; d(m) represents the inner diameter of the pipe; $\mu$ (kg/ms) represents the viscosity coefficient of the fluid; and $\nu$(m$^2$/s) represents the kinematic viscosity coefficient of the fluid.

In the above equation, under the assumption that the density and the viscosity coefficient of the medium are constants, the value of Re is determined dependently on the inner diameter of the pipe through which the medium passes, and the supply rate of the medium. Therefore, for example, by providing a channel in the cell culture section, and appropriately selecting the inner diameter of the channel and the flow rate of the medium transferred into the channel, the medium can be transferred into the cell culture section under laminar flow conditions.

The size of the channel (groove 11) is not limited, and for example, the length of the longest portion of the cross-section vertical to the length direction of the channel may be within the range of, for example, 0.1 mm to 300 mm. When the channel has a cylindrical shape, the inner diameter of the channel may be within the range of 0.1 mm to 300 mm. When the channel has a square tube shape, the height and/or width of the channel may be within the range of 0.1 mm to 300 mm. The length of the longest portion of the cross-section vertical to the length direction of the channel is preferably within the range of 1 mm to 100 mm, and when the channel has a cylindrical shape, the inner diameter of the channel is preferably within the range of 1 mm to 100 mm, and when the channel has a square tube shape, the height and/or width of the channel is preferably within the range of 1 mm to 100 mm.

Thus, by sequentially introducing predetermined media into the cell culture device 4 containing seeded cells, cells in a desired differentiation state can be obtained as aggregates having uniform size while the pluripotent stem cells are allowed to differentiate into the cells of interest. For example, by sequentially introducing predetermined media into the cell culture device 4 containing seeded pluripotent stem cells under conditions where the cells are induced to differentiate into pancreatic islet cells, pancreatic islet cells can be obtained as aggregates having uniform size. By controlling the size of the cell aggregates to be constant, uniform differentiation induction efficiency can be realized, and a high cell survival rate can be realized. Moreover, in cases where the differentiation induction is carried out with cells in the state of cell aggregates, the operation to form cell aggregates is unnecessary during the induction process, so that the operation can be simplified. The conditions of the culture of cell aggregates in the wells 17 of the cell culture device 4 are thought to be more similar to the conditions of the development in a living body, which is accompanied by formation of a three-dimensional structure. Therefore, it is likely that the present method, compared to conventional techniques, can induce pancreatic islet cells (pseudoislets) having properties more similar to those of pancreatic islets in a living body.

For collection of the cell aggregates from the cell culture device 4, for example, the cell culture device 4 may be turned upside down to drop the aggregates from the wells 17 to the groove 11, and a medium may then be introduced from the inlet end. By this, the medium containing the cell aggregates can be collected from the discharge end. Alternatively, the cell aggregates may be collected from the wells 17 after removing the cover 10 from the main body 9.

In the above example, the cell suspension was directly introduced into the cell culture device 4. Alternatively, the bag 2 may be used. By connecting the other end of the tube 7 to the inlet end of the cell culture device 4, placing the cell suspension in the bag 2, and operating the pump 3, the cell suspension can be sucked from the bag 2 and discharged toward the cell culture device 4. By introduction of the cell suspension into the cell culture device 4 through the tube 7, the cells are seeded in the cell culture device 4. The introduction of the cell suspension is carried out such that the cell suspension is spread over the entire groove 11 of the cell culture device 4 in, for example, about 2 seconds. After the introduction of the cell suspension into the cell culture device 4, the operation of the pump 3 is stopped to leave the cells to stand.

In the above embodiment, the well 17 has a circular shape in its planar view, and a semielliptical shape in its longitudinal section. However, for example, the following modifications are possible.

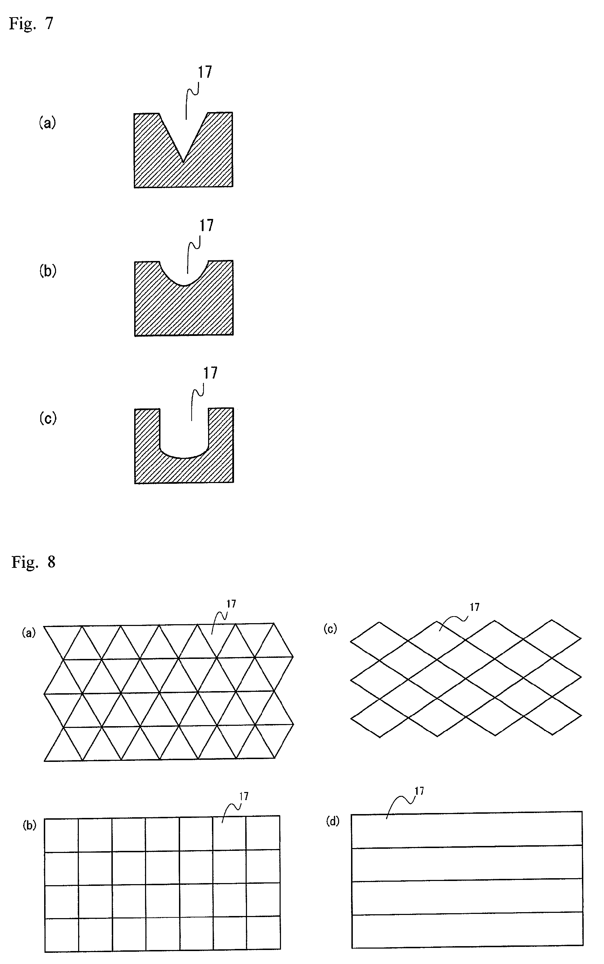

Fig. 7 is a diagram showing examples of the cross-sectional shape of the well. In the example shown in Fig. 7(a), the well 17 has a triangular cross-sectional shape. In this case, the well 17 has a conical shape as a whole. In the example shown in Fig. 7(b), the well 17 has a semicircular cross-sectional shape. In this case, the well 17 has a hemispherical shape as a whole. In the example shown in Fig. 7(c), the well has a rectangular cross-sectional shape having round corners in the bottom portion. In this case, the well 17 has a cylindrical shape having a bowl-shaped bottom portion, as a whole.

The cross-sectional shape of the well 17 is not limited to the examples shown in Fig. 7, and preferably gets thinner toward the bottom. This is because such a shape easily allows the cells to gather in the bottom portion of the well 17, and to form an aggregate.

Fig. 8 shows examples of the top view of the wells on the bottom surface of the groove. In Fig. 8, modification examples of the shape of the well 17 are shown. In the example shown in Fig. 8(a), each well 17 has a triangular shape in its planar view. In the example shown in Fig. 8(b), each well 17 has a square shape in its planar view. In the example shown in Fig. 8(c), each well 17 has a diamond shape in its planar view. In the examples shown in Fig. 8(a), (b), and (c), wells 17 are tightly placed between adjacent wells 17 on the groove 11. In these examples, each well 17 is in contact with adjacent wells 17, and, unlike the case of the wells 17 having a circular shape shown in Fig. 3, there are no flat sections like the flat sections 20 shown in Fig. 6(a) between the wells 17. Therefore, the cells are less likely to be cultured in areas other than the wells 17, and uniform cell clusters are therefore likely to be formed. The length of each side of the well 17 is preferably 100 $\mu$m to 5 mm. The height (depth) of the well 17 is preferably 100 $\mu$m to 5 mm. The length of each side of the well 17 is an example of the size of the well 17. The shape of the well 17 is not limited to the above-described examples, and may be a different shape in which the flat sections in the vicinities of the wells 17 are small or absent. The length of each side of the well 17 is an example of the width of the well 17.

In the example shown in Fig. 8(d), the well 17 has a rectangular shape, and the wells 17 are tightly arranged on the bottom surface of the groove 11 without forming gaps. In cases where the wells 17 are constituted like this, cylindrical cell clusters are formed. The length of each short side of the rectangular well 17 is preferably 100 $\mu$m to 5 mm. The height (depth) of the well 17 is preferably 100 $\mu$m to 5 mm. The length of each short side of the rectangular well 17 is an example of the width of the well 17. Each cell cluster may have a cylindrical shape as long as the distance from the side surface of the cylinder to the center of the aggregate allows diffusion of oxygen and nutrients necessary for the survival of the cells.

In the embodiments described above, the channel has a shape like the groove 11 shown in Fig. 3. However, the following modifications are possible.

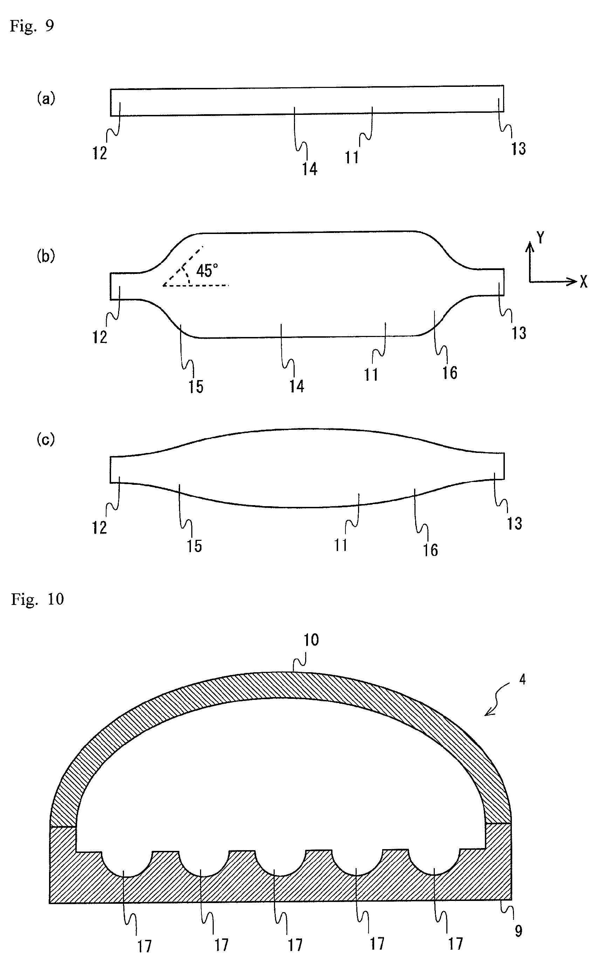

Fig. 9 is a diagram showing examples of the shape of the channel. Fig. 9 shows examples of the top view of the middle section 14. The shape of the channel in the middle section 14 is not limited to the examples shown in Fig. 3, and the channel may have a different shape.

As shown in Fig. 9(a), the width of the middle section 14 may be the same as the width of the one end section 12 and the other end section 13, and the taper sections 15 and 16 may be absent. In such a case, the medium introduced into the inlet end can be securely spread to both side-wall surfaces of the middle section 14 even at a flow rate higher than that in a channel like the one shown in Fig. 3. As shown in Fig. 9(b), the taper section 15 is formed such that the angle formed by each tangent plane (contact surface) of the two side-wall surfaces of the taper section 15 and the direction from the inlet end toward the discharge end (X-direction) is not more than 45°. Preferably, the angle formed by the tangent plane (contact surface) at any position of the two side-wall surfaces of the taper section 15 and the X-direction is not more than 45°. In such a case, the medium introduced from the inlet end can be spread to the ends (side-wall surfaces) of the taper section 15. In these cases, the shape of the side wall surfaces of the taper section 15 may be either flat or curved. As shown in Fig. 9(c), the middle section 14 may be absent, and the taper section 15 and the taper section 16 may be continuous with each other.

The cover 10 described in the above embodiments may be modified as follows.

Fig. 10 is a cross-sectional view of the cell culture device showing a modified example of the cover. The cross-sectional view of the cell culture device 4 shown in Fig. 10 is a cross-sectional view at the position corresponding to the position for the cross-sectional view shown in Fig. 5. The covers 10 of the cell culture devices 4 shown in Fig. 5 and the like are flat, but the cover 10 may be in the shape of a dome inflated outwardly as shown in Fig. 10. The device may be used in a state where the cover 10 is removed.

As long as the medium uniformly flows through the groove 11, a groove connecting the one end section 12 to the outside may be formed, and the tube 7 may be connected to the connection portion between this groove and the outside. Similarly, a groove connecting the other end section 13 to the outside may be formed, and the tube 8 may be connected to the connection portion between this groove and the outside. In such a case, the cover 10 is not provided with the first hole 18 and the second hole.

The cover 10 may be formed such that the taper section 15, middle section 14, and taper section 16 can be covered therewith, and may have a constitution in which the one end section 12 and the other end section 13 are exposed. In such a case, the cover 10 is not provided with the first hole 18 and the second hole 19, and the tube 7 and the tube 8 are connected to the one end section 12 as the inlet end, and the other end section 13 as the discharge end, respectively. In such a case, the one end section 12 and the other end section 13 are formed such that they fit the external shapes of the tube 7 and the tube 8, respectively, so that the medium does not leak to the outside when they are connected to the tubes.

The bag 2 and the tube 6 in the above embodiments may be modified as follows.

In the middle of the tube 6, a switching valve capable of switching the connection of the tube may be provided, and bags 2 containing a plurality of kinds of media may be connected to the switching valve. In such a case, medium replacement can be easily carried out by operating the switching valve.

In the cell culture system 1 as described above, cells are seeded in the cell culture device 4, and a medium is introduced to the device to perform cell culture. In the cell culture device 4 of the cell culture system 1, a channel having a plurality of wells 17 is formed, and the medium can be easily introduced into, and discharged from, the cell culture device 4. Since the cell culture system 1 enables easy introduction and discharge of the medium, the medium can be easily replaced without performing centrifugation or the like.

In the cell culture device 4, a plurality of wells 17 having the same size are provided such that the wells are adjacent to each other, and such that the area of the flat sections 20 on the bottom surface of the groove 11 is small. Since the cell culture device 4 is provided with the wells 17 having the same size, cell aggregates having uniform size can be formed. Since the flat sections 20 on the bottom surface of the groove 11 are small, cell aggregates are unlikely to be formed in areas other than the inside of the wells 17, and cell aggregates having more uniform size can be formed.

The cell culture device 4 described in the above embodiment can be modified as follows.

Fig. 12 is a diagram showing a modification example of the cell culture device. Fig. 12 is a top view of a modification example of the main body 9 of the cell culture device 4. In the cell culture device 4 in Fig. 3, wells 17 are formed in the taper section 15, middle section 14, and taper section 16. However, in the cell culture device 4 in Fig. 12, wells 17 are not formed in the taper section 15 and the taper section 16, while wells 17 are formed in the middle section 14. Since the width of each of the taper section 15 and the taper section 16 is different from the width of the middle section 14, the medium may flow at different rates when the medium is allowed to flow through the cell culture device 4. In view of this, in cases where wells 17 are not formed in the taper section 15 and the taper section 16, while wells 17 are formed in the middle section 14, the culture conditions in the wells 17 can be more uniform. Since the width of the middle section 14 is uniform, the medium flows at a uniform rate in the middle section 14. In the middle section 14, wells 17 do not need to be formed in the areas near the taper section 15 or the taper section 16.

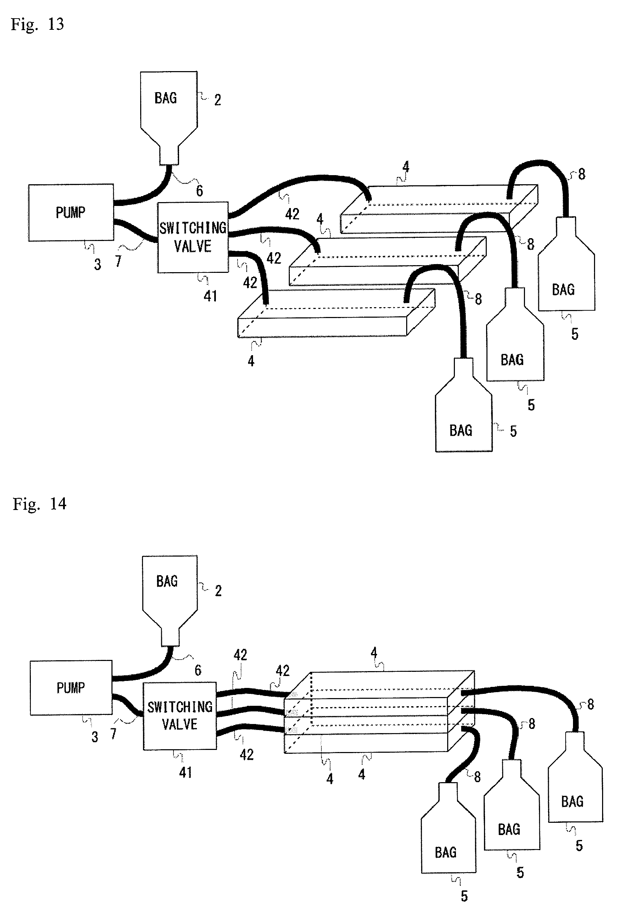

Fig. 13 is a diagram showing alternative example 1 of the cell culture system of this embodiment. The cell culture system 1 in Fig. 13 comprises a bag 2; a pump 3; a switching valve 41 as a branching section; a plurality of cell culture devices 4; and a plurality of waste liquid storage bags 5. The bag 2 is connected to the pump 3 through a tube 6; the pump 3 is connected to the switching valve 41 through a tube 7; the switching valve 41 is connected to the cell culture devices 4 through tubes 42; and the cell culture devices 4 are connected to the bags 5 through tubes 8. By the switching valve 41, the destination of the flow of a liquid such as a medium flowing from the tube 7 is switched. The tubes 8 may also be connected to a single bag 5. Here, the cell culture devices 4 are arranged in parallel in the horizontal direction. By such arrangement, a larger amount of cells can be cultured at once. Depending on culture conditions, in cases where there is no need to allow the medium to flow constantly through each cell culture device 4, the cell culture device(s) 4 through which the medium flows may be switched by the switching valve 41. By this, a large amount of cells can be efficiently cultured at once. Although three cell culture devices 4 are arranged in Fig. 13, the number of cell culture devices 4 is not limited to three.

Fig. 14 is a diagram showing alternative example 2 of the cell culture system of this embodiment. The cell culture system 1 in Fig. 14 comprises a bag 2; a pump 3; a switching valve 41 as a branching section; a plurality of cell culture devices 4; and a plurality of waste liquid storage bags 5. Here, the cell culture devices 4 are arranged such that the devices are vertically stacked. By such arrangement, a larger amount of cells can be cultured at once. Here, holes for connecting the cell culture devices 4 to the tubes 42 or the tubes 8 are arranged on side surfaces of the cell culture devices 4. The arrangement of the holes on the side surfaces allows the culture devices 4 to be stacked in the vertical direction. Depending on culture conditions, in cases where there is no need to allow the medium to flow constantly through each cell culture device 4 (that is, in cases where the medium may be intermittently supplied), the cell culture device(s) 4 through which the medium flows may be switched by the switching valve 41. By this, a large amount of cells can be efficiently cultured at once. Although three cell culture devices 4 are arranged in Fig. 14, the number of cell culture devices 4 is not limited to three.

By combination of the alternative example 1 and alternative example 2, cell culture devices 4 may be arranged both horizontally and vertically. By this, a larger amount of cells can be cultured.

In this embodiment and the alternative examples, the height of the wall surfaces of the taper section 15, middle section 14, and taper section 16 is preferably 10 $\mu$m to 100 mm. The height is more preferably 100 $\mu$m to 10 mm. The height is still more preferably 200 $\mu$m to 1 mm. In cases where the wall surfaces are too low, it is difficult to spread fresh medium over the wells 17. Moreover, in cases where the wall surfaces are too low, the flow rate of the medium is high, and the cells may therefore escape from the wells 17. On the other hand, in cases where the wall surfaces are too high, medium unnecessary for culturing the cells in the wells 17 flows, leading to waste of the medium. Moreover, in cases where the wall surfaces are too high, the flow rate of the medium cannot be easily kept constant, so that maintenance of the laminar flow is difficult. Thus, the height of the wall surfaces is preferably within the range described in the present description.

The rate and the frequency of introduction of the medium (culture medium) into the wells 17, in which the cells are cultured, need to be controlled depending on the nutrient consumption by the cells and the rate of accumulation of wastes. The height of the wall surfaces (that is, the height of the channel through which the medium flows) needs to be low to an extent that the resulting high flow rate does not affect cell aggregates in the wells. On the other hand, the height of the wall surfaces needs to be high to an extent that the medium is not wasted when the medium is replaced with another type of medium.

The cell culture system 1 can be used for any cell culture where formation of cell aggregates of uniform size is required. Examples of cells to be cultured in cell aggregates include embryoid bodies, hepatocytes, cardiomyocytes, neural cells, kidney cells, hepatocytes, chondrocytes, retina cells, trichocysts as well as pancreatic islet cells. In addition to pancreatic islet cells, the cell culture system can be efficiently used for culturing embryoid bodies, hepatocytes, cardiomyocytes, and neural cells.

The constitutions of the embodiments and modification examples described above may be carried out in combination whenever possible.

DESCRIPTION OF SYMBOLS

1. Cell culture system
2. Bag
3. Pump
4. Cell culture device (Cell culture vessel)
5. Bag
6. Tube
7. Tube
8. Tube

**Claims**

**1.** A cell culture device comprising:

a channel having an inlet end for the introduction of a liquid and a bottom surface, wherein said channel is configured for the flow through of said liquid; and
a plurality of recesses for containing cells, which are formed on said bottom surface of said channel;

wherein said plurality of recesses are closely arranged on said bottom surface of said channel.

**2.** The cell culture device according to claim 1, wherein said channel has a discharge end for discharging said liquid;
said channel comprises a first area the width of which increases from said inlet end to a predetermined length;
said first area has two side-wall surfaces which are perpendicular to said bottom surface of said channel; and
the angle formed by each tangent plane of said two side-wall surfaces and the direction from said inlet end toward said discharge end is not more than 45°.

**3.** The cell culture device according to claim 1 or 2, wherein
the area of said plurality of recesses on said bottom surface of said channel is not less than half of the area of said bottom surface.

**4.** The cell culture device according to any one of claims 1 to 3, wherein each of said recces has a rim and the rim of said recess is chamfered.

**5.** The cell culture device according to any one of claims 1 to 4, wherein
the width of said recess is 100 $\mu$m to 5 mm; and the height of said recess is 100 $\mu$m to 5 mm.

**6.** A cell culture system comprising:

the cell culture device according to any one of claims 1 to 5;
a feeding device for adjusting the amount of a liquid introduced to said cell culture device; and
a storage device for storing cells and a liquid discharged from said cell culture device.

**7.** A method for culturing cells, said method comprising the steps of:

providing a cell culture device according to any one of claims 1 to 5;
introducing a medium at the inlet end of the channel of said device whereby said medium flows through said channel;
seeding cells in the recesses of said device to prepare aggregates having a predetermined size; and
culturing said aggregates in said medium introduced from said inlet end.

**8.** The method according to claim 7, wherein the flow rate of said liquid introduced to said channel is not more than 10 cm/s.

**9.** The method according to claim 7 or 8, wherein said culturing is carried out using a laminar flow of said medium in said channel.

**10.** The method according to any one of claims 7 to 9, wherein said cells seeded in said recesses are pluripotent stem cells.

**11.** The method according to claim 10, wherein said pluripotent stem cells are induced to differentiate into pancreatic islet cells.

Fig. 1

Fig. 2

Fig. 3

Fig. 4

Fig. 5

Fig. 6

(a)

(b)

Fig. 7

(a)

17

(b)

17

(c)

17

Fig. 8

(a)

17

(c)

17

(b)

17

(d)

17

Fig. 9

(a)

(b)

45°

(c)

Fig. 10

Fig. 11

20 17 TRIANGULAR LATTICE LATTICE SPACING    LATTICE POINT

Fig. 12

Fig. 13

Fig. 14

Europäisches
Patentamt

European
Patent Office

Office européen
des brevets

# EUROPEAN SEARCH REPORT

**Application Number**

EP 16 16 5441

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2013/261021 A1 (BOCCHI ET AL.) 3 October 2013 (2013-10-03) * figures 1, 3, 8 * * paragraphs [0125], [0134]; claims 44,46,48 * | 1,3,6,7 | INV. C12M1/32 C12M1/00 |
| X | US 2011/183312 A1 (YANYI HUANG) 28 July 2011 (2011-07-28) | 1-6 | |
| Y | * paragraphs [0001], [0004], [0005], [0038], [0041], [0054], [0057], [0062], [0063]; figures 1,2 * | 7-11 | |
| Y | WO 2014/165273 A1 (INNOVATIVE SURFACE TECHNOLOGIES INC.) 9 October 2014 (2014-10-09) * claims 11,23; figures 1,3,4 * * page 22, line 6 - line 12 * * page 23, line 3 - line 7 * * page 23, line 29 - page 24, line 12 * | 7-11 | |
| Y,D | THOMAS C. SCHULZ: "A Scalable System for Production of Functional Pancreatic Progenitors from Human Embryonic Stem Cells", PLOS ONE, vol. 7, no. 5, 1 May 2012 (2012-05-01), page E37004, XP55059404, * "Materials and Methods"; page 12 * | 7-11 | **TECHNICAL FIELDS SEARCHED (IPC)** C12M |
| X | EP 1 379 622 B1 (EVOTEC AG) 12 November 2008 (2008-11-12) * figures 1-4 * * paragraphs [0001], [0009], [0011], [0015], [0024] * | 1,3,6 | |

-/--

The present search report has been drawn up for all claims

| Place of search | Date of completion of the search | Examiner |
|---|---|---|
| Berlin | 6 September 2016 | Alvarez Alvarez, C |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

 

& : member of the same patent family, corresponding document

EPO FORM 1503 03.82 (P04C01)

Europäisches
Patentamt

European
Patent Office

Office européen
des brevets

**EUROPEAN SEARCH REPORT**

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | CA 2 098 331 A1 (LAWRENCE ET AL.) 15 December 1994 (1994-12-15) * page 3, paragraph 3; figures 1,2,6 * * page 7, paragraphs 3,5,6 * ----- | 1,6 | |

TECHNICAL FIELDS
SEARCHED      (IPC)

The present search report has been drawn up for all claims

| Place of search | Date of completion of the search | Examiner |
|---|---|---|
| Berlin | 6 September 2016 | Alvarez Alvarez, C |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

                                                   
& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 16 16 5441

This annex lists the patent family members relating to the patent documents cited in the above-mentioned European search report.
The members are as contained in the European Patent Office EDP file on
The European Patent Office is in no way liable for these particulars which are merely given for the purpose of information.

06-09-2016

| Patent document cited in search report | | Publication date | Patent family member(s) | | Publication date |
|---|---|---|---|---|---|
| US 2013261021 | A1 | 03-10-2013 | CN | 103392124 A | 13-11-2013 |
| | | | CN | 105842435 A | 10-08-2016 |
| | | | EP | 2646798 A1 | 09-10-2013 |
| | | | EP | 3035031 A1 | 22-06-2016 |
| | | | US | 2013261021 A1 | 03-10-2013 |
| | | | US | 2016178502 A1 | 23-06-2016 |
| | | | WO | 2012072822 A1 | 07-06-2012 |
| US 2011183312 | A1 | 28-07-2011 | CN | 101827931 A | 08-09-2010 |
| | | | US | 2011183312 A1 | 28-07-2011 |
| | | | WO | 2010023497 A1 | 04-03-2010 |
| WO 2014165273 | A1 | 09-10-2014 | US | 2016032238 A1 | 04-02-2016 |
| | | | WO | 2014165273 A1 | 09-10-2014 |
| EP 1379622 | B1 | 12-11-2008 | AT | 414136 T | 15-11-2008 |
| | | | DE | 10118905 A1 | 24-10-2002 |
| | | | EP | 1379622 A2 | 14-01-2004 |
| | | | WO | 02099033 A2 | 12-12-2002 |
| CA 2098331 | A1 | 15-12-1994 | NONE | | |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 8859286 B
- JP 2011115161 A

- JP 5039715 B
- JP 2007135593 A

**Non-patent literature cited in the description**

- *DIABETES,* August 2012, vol. 61, 2016-2029
- *PLoS ONE,* May 2012, (7), 37004

- **THOMAS C. SCHULZ ; FELICIA W PAGLIUCA et al.** *Cell,* October 2014, vol. 159, 428-439
- *PLoS ONE,* 07 May 2012, 37004
- *Cell,* October 2014, vol. 159, 428-439